# EUROPEAN PATENT SPECIFICATION

(11) **EP 1 292 555 B1**
(45) Date of publication and mention of the grant of the patent: **03.03.2004**
(21) Application number: 01948513.5
(22) Date of filing: 20.06.2001
(51) Int. Cl.: C07C 1/207, C07C 29/141, C07C 29/143, C07C 67/31, C07D 307/32

(54) **CATALYTIC HYDROGENATION OF KETONES AND ALDEHYDES**
KATALYTISCHE HYDRIERUNG VON KETONEN UND ALDEHYDEN
HYDROGENATION CATALYTIQUE DE CETONES ET D'ALDEHYDES

(30) Priority: 20.06.2000 US 212497 P
(43) Date of publication of application: 19.03.2003
(73) Proprietor: E.I. DUPONT DE NEMOURS AND COMPANY, Wilmington, Delaware 19898 (US)
(72) Inventor: BULLOCK, R., Morris, Wading River, NY 11792 (US); FAGAN, Paul, Joseph, Wilmington, DE 19803 (US); HAUPTMAN, Elisabeth, M., Princeton NJ 08540 (US); SCHLAF, Marcel, Guelph, Ontario N1H 3S5 (CA)
(74) Representative: Carpmaels & Ransford
(86) International application number: PCT/US2001/019680
(87) International publication number: WO 2001/098238

(56) References cited:
- US-A- 4 418 227
- R.A. SANCHEZ-DELGADO, R A ET AL: "Homogeneous hydrogenation of aldehydes to alcohols with ruthenium complex catalysts" JOURNAL OF ORGANOMETALLIC CHEMISTRY, vol. 209, no. 1, 7 April 1981 (1981-04-07), pages 77-83, XP002042304 Elsevier Sequoia, Lausanne, CH ISSN: 0022-328X
- K. HATA, ET AL.: "Vapour phase catalytic hydrogenation of organic compounds using stabilised nickel catalyst" BULLETIN OF THE CHEMICAL SOCIETY OF JAPAN, vol. 31, no. 6, September 1958 (1958-09), pages 775-776, XP002193730 Japan Publications Trading Co., Tokyo, JP ISSN: 0009-2673

## Description

### FIELD OF INVENTION

This invention relates to a method of reducing ketones and aldehydes to the corresponding alcohol or alkyl group, using H₂ gas as the stoichiometric reductant, and organometallic ruthenium complexes as the catalysts.

### BACKGROUND

The homogeneous catalyzed reduction of ketones and aldehydes to various products is an important synthetic reaction in industry. Ruthenium complexes are well known catalysts for the reduction of ketones and aldehydes to various end products. Often phosphorus-containing ligands are used to complex the ruthenium (U.S. Patent No. 5,614,641, U.S. Patent No. 4,418,227, and Ohkuma, et al., *J*. *Amer*. *Chem*. *Soc*., 1998, 120, 1086). Typically, phosphorus-containing ligands are expensive, difficult to make and handle, and sensitive to oxygen.

Chinn, et al, (*Organometallics* 1989, *8*, 1824-1826) described the synthesis and spectroscopic properties of the unstable dihydrogen complex [Cp*Ru(CO)₂(H₂)]⁺ which was found to decompose to {[Cp*Ru(CO)₂]₂(µ-H)}⁺OTf⁻, where Cp* indicates a η⁵-C₅Me₅ group. The dihydrogen complex is synthesized under mild conditions, has no need for oxygen sensitive ligands (e.g., triaryl phosphines, trialkyl phosphines) or nitrogen-based ligands, and is tolerant to acid and water. However, no mention is made of the catalytic activity of any of these complexes.

### SUMMARY OF THE INVENTION

The invention is directed to a process for the reduction of a ketone or aldehyde, comprising contacting a compound of the formula R¹-C(=O)-R² with hydrogen in the presence of a catalytically effective amount of a catalyst precursor having the formula {[CpM(CO)₂]₂(µ-H)}⁺Q⁻, wherein M is selected from the group consisting of Ru and Fe, R¹ is selected from the group consisting of hydrogen, substituted and unsubstituted alkyl and aryl groups, R² is selected from the group consisting of substituted and unsubstituted alkyl and aryl groups; Cp is η⁵-C₅R₅ wherein R is selected from the group consisting of hydrogen, substituted and unsubstituted C₁-C₁₈ alkyl and aryl groups; and Q- is a non-coordinating or weakly coordinating non-reactive anion. R¹ and R² may form a ring together.

Preferably, M is Ru, Q⁻ is OSO₂CF₃⁻, R is selected from the group consisting of hydrogen and methyl, and the compound of the formula R¹-C(=O)-R² is selected from the group consisting of cyclooctanone, levulinic acid, levulinic acid methyl ester, benzylacetone, 3-heptanone, 4-methoxyacetoacetate, 3-pentanone and propanal.

Also preferably R¹ is selected from the group consisting of hydrogen and substituted and unsubstituted alkyl groups, R² is selected from the groups consisting of substituted and unsubstituted alkyl groups, and the reduction product is an alcohol.

In the case where R¹ and R² have not formed a ring, the process can further comprise the cyclizing of the alcohol to form the corresponding lactone.

The invention is further directed to a process to reduce a ketone or aldehyde to form a compound of the formula R¹-CH₂-R², comprising contacting a compound of the formula R¹-C(=O)-R² with hydrogen in the presence of a catalytically effective amount of a complex having the formula {[CpRu(CO)₂]₂(µ-H)}⁺Q⁻, wherein R¹ is selected from the group consisting of hydrogen, substituted and unsubstituted alkyl and aryl groups; R² is selected from the group consisting of unsubstituted and substituted aryl groups, Cp is η⁵-C₅R₅ wherein R is selected from the group consisting of hydrogen, and unsubstituted and substituted C₁-C₁₈ alkyl and aryl groups; and Q⁻ is a non-coordinating or weakly coordinating non-reactive anion. R¹ and R² may form a ring together.

Preferably Q⁻ is OSO₂CF₃⁻ and R is selected from the group consisting of hydrogen and methyl, and the compound of formula R¹-C(=O)-R² is selected from the group consisting of 1-acetonaphthalene and acetophenone.

### DETAILED DESCRIPTION OF THE INVENTION

In the present description, "alkyl" means an alkyl group containing up to 18 carbon atoms. Common examples of such alkyl groups include methyl, ethyl, propyl, isopropyl, butyl, s-butyl, isobutyl, pentyl, neopentyl, hexyl, heptyl, isoheptyl, 2-ethylhexyl, cyclohexyl and octyl. The alkyl group may be linear, branched, or cyclic.

By "substituted" is meant the addition of one or more substituent groups to a compound or functional group. Said substituent groups do not cause the compound to be unstable or unsuitable for use in an intended reaction, and are inert under reaction conditions. Substituent groups which are generally useful include nitrile, ether, ester, halo, amino (including primary, secondary and tertiary amino), hydroxy, oxo, vinylidene or substituted vinylidene, carboxyl, silyl or substituted silyl, nitro, sulfinyl, and thioether. Highly basic substituents are generally not suitable in the process of present invention unless previously piotonated with acid or protected by a suitable protecting group.

By "aryl" is meant an aromatic carbocyclic group having a single ring (e.g., phenyl), multiple rings (e.g., biphenyl), or multiple condensed rings in which at least one is aromatic, (e.g., 1,2,3,4-tetrahydronaphthyl, naphthyl, anthryl, or phenanthryl), which may be mono-, di-, or trisubstituted with halogen, lower alkyl, lower alkoxy, lower alkylthio, trifluoromethyl, lower acyloay, aryl, heteroaryl, or hydroxy groups. By "aryl" is also meant heteroaryl groups where heteroaryl is defined as 5-, 6-, or 7-membered aromatic ring systems having at least one heteroatom selected from the group consisting of nitrogen, oxygen and sulfur. Examples of heteroaryl groups are pyridyl, pyrimidinyl, pyrrolyl, pyrazolyl, pyrazinyl, pyridazinyl, oxazolyl, furanyl, quinolinyl, isoquinolinyl, thiazolyl, and thienyl. Said heteroaryl groups may contain substituent groups including halogen, lower alkyl, lower alkoxy, lower alkylthio, trifluoromethyl, lower acyloxy, aryl, heteroaryl, and hydroxy.

The following definitions are used herein:
Ar'=3,5-bis(trifluoromethyl)phenyl
Cp* =η⁵-C₅Me₅
HOTf=CF₃SO₃H
OTf⁻ = OSO₂CF₃⁻
OTf = OSO₂CF₃

The present invention provides a process to prepare an alcohol via the hydrogenation of a ketone or aldehyde by contacting a compound of the formula R¹-C(=O)-R² with hydrogen in the presence of a catalytically effective amount of a catalyst precursor, to form an alcohol, wherein R¹ is selected from the group consisting of hydrogen, substituted and unsubstituted alkyl and aryl groups; R² is selected from the group consisting of substituted and unsubstituted alkyl and aryl groups; and R¹ and R² may form a ring together. The catalyst precursor is of the formula {[CpM(CO)₂]₂(µ-H)}⁺Q⁻ where Q⁻ is a non-coordinating or weakly coordinating non-reactive anion, M is selected from the group consisting of Fe and Ru, and Cp is η⁵-C₅R₅ wherein R is selected from the group consisting of hydrogen and substituted and unsubstituted C₁-C₁₈ alkyl and aryl groups. Preferably Q⁻ is OSO₂CF₃⁻, R is hydrogen or methyl and M is Ru.

Preferred compounds of formula R¹-C(=O)-R² include cyclooctanone, levulinic acid, levulinic acid methyl ester, benzylacetone, 3-heptanone, 4-methoayaoetoacetate, 3-pentanone, and propanal.

By "alcohol" is meant a compound containing an alcohol functionality. By "ketone" it is meant a compound containing a ketone functionality. By "aldehyde" it is meant a compound containing an aldehyde functionality.

The invention also provides a process to cyclize the alcohol to form a corresponding lactone. This may occur spontaneously under the reaction conditions when the keto group of an ester or carboxylic acid is in the γ, δ or higher position relative to the carbonyl group of the reacting aldehyde or ketone, forming a 5-, 6- or higher membered ring, and eliminating an alcohol in the case of an ester, and water in the case of a carboxylic acid. Two examples of this process are illustrated below. The preferred compound for this embodiment is levulinic acid.

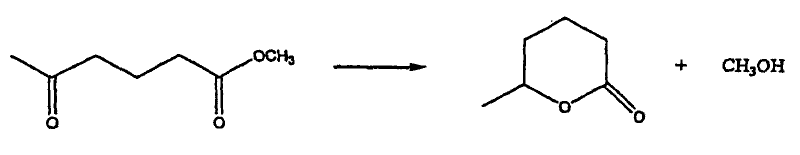

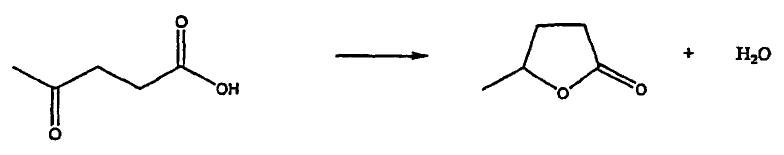

The invention also provides a process to reduce a ketone or aldehyde to form a compound of the formula R¹-CH₂-R², comprising contacting a compound of the formula R¹-C(=O)-R² with hydrogen in the presence of a catalytically effective amount of a catalyst precursor, wherein R¹ is selected from the group consisting of hydrogen, substituted and unsubstituted alkyl and aryl groups, R² is selected from the group consisting of unsubstituted and substituted aryl groups, and R¹ and R² may form a ring together. The catalyst precursor is of the formula {[CpM(CO)₂]₂(µ-H)}⁺Q⁻ where Q⁻ is a non-coordinating or weakly coordinating non-reactive anion, M is selected from the group consisting of Fe and Ru, and Cp is η⁵-C₅R₅ wherein R is selected from the group consisting of hydrogen and substituted and unsubstituted C₁-C₁₈ alkyl and aryl groups. Preferably R is hydrogen or methyl, Q⁻ is OSO₂CF₃⁻ and M is Ru. Preferred compounds of the formula R¹-C(=O)-R² include 1-acetonaphthalene and acetophenone.

When either R¹ or R² is a substituted or unsubstituted aryl group, the reaction will follow the path below under most reaction conditions:

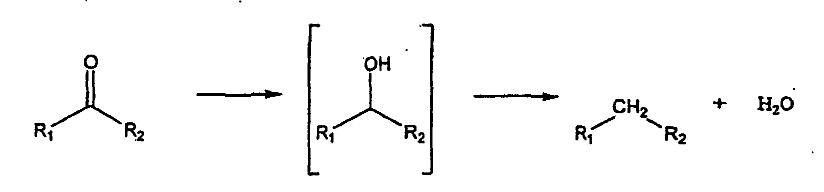

In some instances when an aryl substituent is present, only the alcohol or a mixture of the alcohol and alkyl product can be obtained. When neither of the substituents contains a hydrogen atom on the carbon atom that is alpha to the oxo group only the alcohol is obtained.

The catalytically active species for both processes is believed to be [CpM(CO)₂]H in combination with one or both of the transient complexes [CpM(CO)₂(η²-H₂)]⁺ and [CpM(CO)₂]⁺, all of which are generated under the reaction conditions. Any synthetic route may be used that leads to the same reactive species, such as use of CpM(CO)₂OQ, [CpM(CO)₂]₂ and H⁺Q⁻, or CpM(CO)₂H and H⁺Q⁻ as catalyst precursors.

The catalyst precursor, {[Cp*Ru(CO)₂]₂(µ-H)}⁺OTf⁻, was prepared in the following manner. In a drybox, a flask was charged with Cp*Ru(CO)₂H (2.05 g, 6.986 mmol, prepared as described in Fagan et al., Organomet., 1990, 9, 1843). The Cp*Ru(CO)₂H was dissolved in 2 ml of CH₂Cl₂. To this flask was added triflic acid (310 µl, 3.5 mmol) slowly, resulting in vigorous gas evolution (H₂). Diethyl ether (10 ml) was then added to the flask and a yellow solid precipitated out of solution. The yellow solid was isolated by filtration and rinsed twice with a minimum amount of diethyl ether.

Weakly coordinating anions are known to those skilled in the art. Such anions are often bulky anions, particularly those that may delocalize their negative charge. The coordinating capability of such anions has been discussed in the literature; see, for instance, W. Beck, et al., *Chem*. *Rev*., vol. 88, p. 1405-1421 (1988), and S. H. Strauss, *Chem*. *Rev*., Vol. 93, p. 927-942 (1993). Weakly coordinating anions suitable for the processes of the present invention include OSO₂CF₃⁻ (herein abbreviated as OTf⁻), SO₄²⁻, HSO₄⁻, BF₄⁻, PF₆⁻, SbF₆⁻, BPh₄⁻, and BAr'₄⁻ where Ar' = 3,5-bis(tnfluoromethyl)phenyl. Most preferred is OTf⁻.

The processes of the present invention are carried out under an atmosphere of hydrogen gas or any mixture of hydrogen gas with other gases that do not interfere with the desired reaction, such as nitrogen, helium, neon and argon gases. Other *in situ* sources of hydrogen can also be used. The partial pressure of the hydrogen should be between 14 to 1000 psi (0.1 to 7.0 MPa), preferably 14 to 800 psi (0.1 to 5.5 MPa). The temperature range is 40°C to 120°C, preferably 65°C to 110°C. The temperature and pressure range can vary depending on the selection of solvent, providing the aldehyde or ketone and solvent remain in the liquid phase.

The processes may be run neat or a suitable solvent may be used. Suitable solvents are non-coordinating, non-basic, inert and able to partially dissolve the catalyst precursor under the reaction conditions. The solvents may be deuterated for ease of analysis. Preferred solvents are dichlorobenzene, sulfolane (tetramethylene sulfone; tetrahydrothiophene-1,1-dioxide) and CH₂Cl₂.

### EXAMPLES

Reactions of the following examples were carried out in a reactor composed of a glass vial inside a stainless steel pressure vessel, which can be easily assembled by one skilled in the art.

### EXAMPLE 1

### HYDROGENATION OF 1-ACETONAPHTHALENE TO 1-ETHYLNAPHTHALENE

A 2 mL reactor was charged with 0.150 mL of a 1.00 M solution of 1-acetonaphthalene and 0.050 mL of a 0.030 M solution of [Cp*Ru(CO)₂]₂(µ-H)}⁺OTf⁻ in dichlorobenzene. The reaction was heated at 90°C for 12 hours at 820 psi (5.7 MPa) of hydrogen gas. The reaction was cooled, and analyzed using gas chromatography/mass spectroscopy. Analysis indicated 100% conversion of the 1-acetonaphthalene, with a greater than 90% yield of 1-ethylnaphthalene. Note that in this example, the alcohol itself (presumably initially formed from hydrogenation of the C=O double bond) undergoes a hydrogenation reaction to yield in the final product, 1-ethylnaphthalene.

### EXAMPLE 2

### HYDROGENATION OF LEVULINIC ACID METHYL ESTER TO γ-VALEROLACTONE

A 2 mL reactor was charged with 0.150 mL of a 1.00 M solution of levulinic acid methyl ester in dichlorobenzene, and 0.050 mL of a 0.030 M solution of {[Cp*Ru(CO)₂]₂(µ-H)}⁺OTf⁻ in dichlorobenzene. The reactor was heated to 90°C for 12 hours at 820 psi (5.7 MPa) of hydrogen gas. The reaction was cooled, and analyzed using gas chromatography/mass spectroscopy. Analysis indicated 100% conversion of the levulinic acid methyl ester, with a greater than 90% yield of γ-valerolactone. Note that in this case, the alcohol itself (presumably initially formed from hydrogenation of the C=O double bond) reacts further to reach the final product, a lactone resulting from a ring-closure reaction.

### EXAMPLE 3

### HYDROGENATION OF CYCLOOCTANONE TO CYCLOOCTANOL

A 2 mL reactor was charged with 0.150 mL of a 1.00 M soiution of cyclooctanone and 0.050 mL of a 0.030 M solution of {[Cp*Ru(CO)₂]₂(µ-H)}⁺OTf⁻ in dichlorobenzene. The reaction was heated at 90°C for 12 hours at 820 psi (5.7 MPa) of hydrogen gas. The reaction was cooled, and analyzed using gas chromatography/mass spectroscopy. Analysis indicated 90% conversion of the cyclooctanone with a greater than 80% yield of cyclooctanol.

### EXAMPLE 4

### HYDROGENATION OF BENZYLACETONE TO 4-PHENYL-2-BUTANOL

A 2 mL reactor was charged with 0.150 mL of a 1.00 M solution or benzylacetone and 0.050 mL of a 0.030 M solution of {[Cp*Ru(CO)₂]₂(µ-H)}⁺OTf⁻ in dichlorobenzene. The reaction was heated at 90°C for 12 hours at 820 psi (5.7 MPa) of hydrogen gas. The reaction was cooled, and analyzed using gas chromatography/mass spectroscopy. Analysis indicated 100% conversion of the benzylacetane, with a greater than 90% yield of 4-phenyl-2-butanol.

### EXAMPLE 5

### HYDROGENATION OF 3-HEPTANONE TO 3-HEPTANOL

A 2 mL reactor was charged with 0.150 mL of a 1.00 M solution of 3-heptanone and 0.050 mL of a 0.030 M solution of {[Cp*Ru(CO)₂]₂(µ-H)}⁺OTf⁻ in dichlorobenzene. The reaction was heated at 90°C for 12 hours at 820 psi (5.7 MPa) of hydrogen gas. The reaction was cooled, and analyzed using gas chromatography/mass spectroscopy. Analysis indicated 100% conversion of the 3-heptanone with a greater than 90% yield of 3-heptanol.

### EXAMPLE 6

### HYDROGENATION OF METHYL 4-METHOXYACETOACETATE TO 3-HYDROXY-4-METHOXYBUTYRIC ACID METHYL ESTER.

A 2 mL reactor was charged with 0.150 mL of a 1.00 M solution of 4-methoxyacetoacetate and 0.050 mL of a 0.030 M solution of {[Cp*Ru(CO)₂]₂(µ-H)}⁺OTf⁻ in dichlorobenzene. The reaction was heated at 90°C for 12 hours at 820 psi (5.7 MPa) of hydrogen gas. The reaction was cooled, and analyzed using gas chromatography/mass spectroscopy. Analysis indicated 100% conversion of the 4-methoxyacetoacetate with a greater than 90% yield of 3-hydroxy-4-methoxybutyric acid methyl ester.

### EXAMPLE 7

### HYDROGENATION OF 3-PENTANONE TO 3-PENTANOL

A solution of 3-pentanone (0.14 M) and of {[Cp*Ru(CO)₂]₂(µ-H)}⁺OTf⁻ (0.028 M) in CD₂Cl₂ (0.68 mL) was placed under hydrogen gas (45 psi, 6.31 MPa). The solution was heated for 5 days at 65°C. Analysis by ¹H nuclear magnetic resonance (NMR) spectroscopy indicated a greater than 90% yield of 3-pentanol.

### EXAMPLE 8

### HYDROGENATION OF NEAT 3-PENTANONE TO 3-PENTANOL

A solution of {[Cp*Ru(CO)₂]₂(µ-H)}⁺OTf⁻ (0.0071 M) in 3-pentanone (3.6 mL) was placed under hydrogen (790 psi, 5.4 MPa) and heated for 5 days at 88°C. Analysis by ¹H nuclear magnetic resonance (NMR) spectroscopy indicated a >90% yield of 3-pentanol and a small amount (<10%) of the ether [(C₂H₅)₂CH]₂O, which forms by acid-catalyzed condensation of the alcohol product.

### EXAMPLE 9

### HYDROGENATION OF ACETOPHENONE TO ETHYLBENZENE

A solution of acetophenone (0.19 M) and of {[Cp*Ru(CO)₂]₂(µ-H)}⁺OTf⁻ (0.028 M) in CD₂Cl₂ (0.55 mL) was placed under hydrogen gas (45 psi, 0.31 MPa). The solution was heated for 4 days at 85°C. Analysis by ¹H nuclear magnetic resonance (NMR) spectroscopy indicated about 91% yield of ethylbenzene and 9% of acetophenone remaining.

### EXAMPLE 10

### HYDROGENATION OF PROPANAL TO n-PROPYL ALCOHOL

A solution ofpropanal (1.0 M) and of {[Cp*Ru(CO)₂]₂(µ-H)}⁺OTf⁻ (0.005 M) in sulfolane (50 mL) was placed under hydrogen gas (750 psi, .5.2 MPa). Toluene (0.1 M) was present in the solution as an internal standard for determination of the yield. The solution was heated for 18 hours at 100°C. Analysis by gas chromatography (GC) indicated a 62% yield of n-propanol, with less than 3% propanal remaining.

## Claims

1. A process for the reduction of a ketone or aldehyde, comprising:
contacting a compound of the formula R¹-C(=O)-R² with hydrogen in the presence of a catalytically effective amount of a catalyst precursor having the formula {[CpM(CO)₂]₂(µ-H)}⁺Q⁻;
wherein M is selected from the group consisting of Ru and Fe;
R¹ is selected from the group consisting of hydrogen, substituted and unsubstituted alkyl and aryl groups;
R² is selected from the group consisting of substituted and unsubstituted alkyl and aryl groups, wherein R¹ and R² together can form a ring;
Cp is η⁵-C₅R₅ wherein R is selected from the group consisting of hydrogen, substituted and unsubstituted C₁-C₁₈ alkyl and aryl groups; and
Q⁻ is a non-coordinating or weakly coordinating non-reactive anion.

2. The process according to Claim 1 wherein M is Ru.

3. The process according to Claim 1 wherein R¹ is selected from the group consisting of hydrogen and substituted and unsubstituted alkyl groups, and R² is selected from the groups consisting of substituted and unsubstituted alkyl groups.

4. The process according to Claim 1 wherein R¹ and R² together form a ring.

5. The process according to Claim 1 wherein Q⁻ is OSO₂CF₃⁻ and R is selected from the group consisting of hydrogen and methyl.

6. The process according to Claim 1 wherein the compound of the formula R¹-C(=O)-R² is selected from the group consisting of cyclooctanone, levulinic acid, levulinic acid methyl ester, benzylacetone, 3-heptanone, 4-methoxyacetoacetate, 3-pentanone, propanal, 1-acetonaphthalene and acetophenone.

7. The process according to Claim 1 wherein the process produces an alcohol.

8. The process according to Claim 7 further comprising cyclizing the alcohol to form a corresponding lactone.

9. The process according to Claim 7 wherein the alcohol is levulinic acid or levulinic acid methyl ester.

10. The process according to Claim 1 wherein R² is selected from the group consisting of unsubstituted and substituted aryl groups.

11. The process according to Claim 10 wherein the process forms a compound having the formula R¹-CH₂-R².

12. The process according to Claim 11 wherein R¹ and R² together form a ring.

13. The process according to Claim 12 wherein Q⁻ is OSO₂CF₃⁻ and R is selected from the group consisting of hydrogen and methyl.

14. The process according to Claim 13 wherein the compound of formula R¹-C(=O)-R² is selected from the group consisting of 1-acetonaphthalene and acetophenone.

15. The process according to Claim 1 wherein the process is carried out at a temperature of 65°C to 110°C.

16. The process according to Claim 1 wherein the process is carried out at a pressure of 0.1 MPa to 5.5 MPa.

17. The process according to Claim 1 wherein the process is carried out in a solvent selected from the group consisting of diclilorobenzene, sulfolane or CH₂Cl₂.

## Patentansprüche

1. Verfahren für die Reduzierung eines Ketons oder Aldehyds, umfassend:
in Kontakt bringen eine Verbindung der Formel R¹-C(=O)-R² mit Wasserstoff in der Anwesenheit einer katalytisch wirksamen Menge eines Katalysatorvorläufers mit der Formel {[CpM(CO)₂]₂(µ-H)}⁺Q⁻,
wobei M ausgewählt ist aus der Gruppe, bestehend aus Ru und Fe,
R¹ ist ausgewählt aus der Gruppe, bestehend aus Wasserstoff, substituierten und unsubstituierten Alkyl- und Arylgruppen,
R² ist ausgewählt aus der Gruppe, bestehend aus substituierten und unsubstituierten Alkyl- und Arylgruppen, wobei R¹ und R² zusammen einen Ring bilden können,
Cp ist η⁵-C₅R₅, wobei R ausgewählt ist aus der Gruppe, bestehend aus Wasserstoff, substituierten und unsubstituierten C₁-C₁₈ Alkyl- und Arylgruppen, und
Q⁻ ist ein nicht koordinierendes oder schwach koordinierendes nicht reaktives Anion.

2. Verfahren nach Anspruch 1. **dadurch gekennzeichnet, daß** M Ru ist.

3. Verfahren nach Anspruch 1, **dadurch gekennzeichnet, daß** R¹ ausgewählt ist aus der Gruppe, bestehend aus Wasserstoff und substituierten und unsubstituierten Alkylgruppen, und R² ist ausgewählt aus der Gruppe, bestehend aus substituierten und unsubstituierten Alkylgruppen.

4. Verfahren nach Anspruch 1, **dadurch gekennzeichnet, daß** R¹ und R² zusammen einen Ring bilden.

5. Verfahren nach Anspruch 1, **dadurch gekennzeichnet, daß** Q⁻ OSO₂CF₃⁻ ist, und R ist ausgewählt aus der Gruppe, bestehend aus Wasserstoff und Methyl.

6. Verfahren nach Anspruch 1, **dadurch gekennzeichnet, daß** die Verbindung der Formel R¹-C(=O)-R² ausgewählt ist aus der Gruppe, bestehend aus Cyclooctanon, Lävulinsäure, Lävulinsäuremethylester, Benzylaceton, 3-Heptanon, 4-Methoxyacetoacetat, 3-Pentanon, Propanal, 1-Acetonaphthalin und Acetophenon.

7. Verfahren nach Anspruch 1, **dadurch gekennzeichnet, daß** das Verfahren einen Alkohol erzeugt.

8. Verfahren nach Anspruch 7, ferner umfassend Zyklisieren des Alkohols unter Bilden eines entsprechenden Lactons.

9. Verfahren nach Anspruch 7 **dadurch gekennzeichnet, daß** der Alkohol Lävulinsäure oder Lävulinsäuremethylester ist.

10. Verfahren nach Anspruch 1, **dadurch gekennzeichnet, daß** R² ausgewählt ist aus der Gruppe, bestehend aus unsubstituierten und substituierten Arylgruppen.

11. Verfahren nach Anspruch 10, **dadurch gekennzeichnet, daß** das Verfahren eine Verbindung mit der Formel R¹-CH₂-R² bildet.

12. Verfahren nach Anspruch 11, **dadurch gekennzeichnet, daß** R¹ und R² zusammen einen Ring bilden.

13. Verfahren nach Anspruch 12, **dadurch gekennzeichnet, daß** Q⁻ OSO₂CF₃⁻ ist, und R ist ausgewählt aus der Gruppe, bestehend aus Wasserstoff und Methyl.

14. Verfahren nach Anspruch 13, **dadurch gekennzeichnet, daß** die Verbindung der Formel R¹-C(=O)-R² ausgewählt ist aus der Gruppe, bestehend aus 1-Acetonaphthalin und Acetophenon.

15. Verfahren nach Anspruch 1, **dadurch gekennzeichnet, daß** das Verfahren bei einer Temperatur von 65°C bis 110°C durchgeführt wird.

16. Verfahren nach Anspruch 1, **dadurch gekennzeichnet, daß** das Verfahren bei einem Druck von 0,1 MPa bis 5,5 MPa durchgeführt wird.

17. Verfahren nach Anspruch 1, **dadurch gekennzeichnet, daß** das Verfahren in einem Lösungsmittel, ausgewählt aus der Gruppe, bestehend aus Dichlorbenzol, Sulfolan oder CH₂Cl₂, durchgeführt wird.

## Revendications

1. Procédé pour la réduction d'une cétone ou d'un aldéhyde, comprenant:
la mise en contact d'un composé de formule R¹-C(=O)-R² avec de l'hydrogène en présence d'une quantité catalytiquement efficace d'un précurseur de catalyseur ayant la formule {[CₚM(CO)₂]₂(µ-H)}⁺Q⁻;
dans laquelle M est choisi dans le groupe constitué par Ru et Fe;
R¹ est choisi dans le groupe constitué par l'hydrogène, les groupes alkyles et aryles substitués et non substitués;
R² est choisi dans le groupe constitué par les groupes alkyles et aryles substitués et non substitués, où R¹ et R² peuvent former un cycle ensemble;
Cₚ est η⁵-C₅R₅ où R est choisi dans le groupe constitué par l'hydrogène, les groupes alkyles et aryles en C₁ à C₁₈ substitués et non substitués; et
Q⁻ est un anion non réactif non coordonnant ou faiblement coordonnant.

2. Procédé selon la revendication 1, dans lequel M est Ru.

3. Procédé selon la revendication 1, dans lequel R¹ est choisi dans le groupe constitué par l'hydrogène et les groupes alkyles substitués et non substitués, et R² est choisi parmi les groupes constitués par les groupes alkyles substitués et non substitués.

4. Procédé selon la revendication 1, dans lequel R¹ et R² forment un cycle ensemble.

5. Procédé selon la revendication 1, dans lequel Q⁻ est OSO₂CF₃⁻ et R est choisi dans le groupe constitué par l'hydrogène et un groupe méthyle.

6. Procédé selon la revendication 1, dans lequel le composé de formule R¹-C(=O)-R² est choisi dans le groupe constitué par la cyclooctanone, l'acide lévulinique, l'ester méthylique de l'acide lévulinique, la benzylacétone, la 3-heptanone, le 4-méthoxyacétoacétate, la 3-pentanone, le propanal, le 1-acétonaphtalène et l'acétophénone.

7. Procédé selon la revendication 1, dans lequel le procédé produit un alcool.

8. Procédé selon la revendication 7, comprenant en outre la cyclisation de l'alcool pour former une lactone correspondante.

9. Procédé selon la revendication 7, dans lequel l'alcool est l'acide lévulinique ou l'ester méthylique de l'acide lévulinique.

10. Procédé selon la revendication 1, dans lequel R² est choisi dans le groupe constitué par les groupes aryles non substitués et substitués.

11. Procédé selon la revendication 10, dans lequel le procédé forme un composé ayant la formule R¹-CH₂-R².

12. Procédé selon la revendication 11, dans lequel R¹ et R² forment un cycle ensemble.

13. Procédé selon la revendication 12, dans lequel Q⁻ est OSO₂CF₃⁻ et R est choisi dans le groupe constitué par l'hydrogène et un groupe méthyle.

14. Procédé selon la revendication 13, dans lequel le composé de formule R¹-C(=O)-R² est choisi dans le groupe constitué par le 1-acétonaphtalène et l'acétophénone.

15. Procédé selon la revendication 1, le procédé étant mis en oeuvre à une température de 65°C à 110°C.

16. Procédé selon la revendication 1, le procédé étant mis en oeuvre à une pression de 0,1 MPa à 5,5 MPa.

17. Procédé selon la revendication 1, le procédé étant mis en oeuvre dans un solvant choisi dans le groupe constitué par le dichlorobenzène, le sulfolane ou CH₂Cl₂.
